(19) Europäisches Patentamt
European Patent Office
Office européen des brevets

(11) EP 0 585 165 B1

(12) # FASCICULE DE BREVET EUROPEEN

(45) Date de publication et mention de la délivrance du brevet:
**04.06.1997 Bulletin 1997/23**

(51) Int Cl.6: **C07C 265/16**, C07C 263/10

(21) Numéro de dépôt: **93402065.2**

(22) Date de dépôt: **17.08.1993**

(54) **Procédé de préparation d'isocyanates d'acyle**

Verfahren zur Herstellung von Acylisocyanaten

Process for the preparation of acyl isocyanates

(84) Etats contractants désignés:
**CH DE FR GB IT LI NL**

(30) Priorité: **18.08.1992 FR 9210101**

(43) Date de publication de la demande:
**02.03.1994 Bulletin 1994/09**

(73) Titulaires:
- **SOCIETE NATIONALE DES POUDRES ET EXPLOSIFS**
  **F-75181 Paris Cédex 04 (FR)**
- **Nippon Paint Co., Ltd.**
  **Osaka-shi Osaka-fu (JP)**

(72) Inventeurs:
- **Le Goff, Philippe**
  **F-91140 Villebon sur Yvette (FR)**
- **Dewilde, Danièle**
  **F-77176 Nandy (FR)**
- **Tsuboniwa, Noriyuki**
  **Higashiosaka-shi, Osaka-fu (JP)**
- **Urano, Satoshi**
  **Tsuzuki-gun, Kyoto-fu (JP)**

(74) Mandataire: **Pech, Bernard et al**
**SNPE - Service Propriété Industrielle**
**12, Quai Henri IV**
**75181 Paris Cédex 04 (FR)**

(56) Documents cités:
**EP-A- 0 143 613** **EP-A- 0 202 840**
**US-A- 3 450 747** **US-A- 4 663 473**

- **JOURNAL OF ORGANIC CHEMISTRY vol. 30, no. 12 , Décembre 1965 , EASTON US pages 4306 - 4307 A. J. SPEZIALE 'The Reaction of Oxalyl Chloride with Amides IV. Synthesis of Acyl Isocyanates'**
- **JOURNAL OF ORGANIC CHEMISTRY, vol. 30, no. 12, décembre 1965, Easron, US, pages 4306 - 4307, A. J. SPEZIALE: "The Reaction of Oxalyl Chloride with Amides IV. Synthesis of Acyl Isocyanates"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne un nouveau procédé de préparation d'isocyanates d'acyle. Plus particulièrement elle concerne un procédé de préparation d'isocyanates d'acyle au moyen de chlorure d'oxalyle.

Les isocyanates d'acyle sont en raison de leur excellente réactivité des intermédiaires très utiles pour former des urées, des carbamates et des polymères.

Le premier procédé de préparation des isocyanates d'acyle consistait à faire réagir un chlorure d'acyle avec le cyanate d'argent (J. Am. Chem. Soc. 62, 1595 (1940). Malheureusement ce procédé ne peut être utilisé industriellement en raison du coût excessif du cyanate d'argent.

Selon un autre procédé on remplace le cyanate d'argent par de l'acide isocyanique (brevet US n° 3 155 700). Mais cet acide est très instable et très difficile à préparer par décomposition de l'acide isocyanurique à une température très élevée telle que 620°C.

De 1962 à 1965, A.J. Speziale et al ont développé un procédé de préparation d'isocyanates d'acyle à partir d'amides par réaction avec du chlorure d'oxalyle (J. Org. Chem., 27, 3742 (1962), 28, 1805 (1963), 30, 4306 (1965). Malheureusement ce procédé donne des résultats très variables selon les amides de départ utilisés, en particulier les rendements en isocyanates d'acyle à partir d'amides aliphatiques sont très faibles. De plus la réaction est généralement très fortement exothermique et rend particulièrement délicate l'obtention des isocyanates thermosensibles. Le contrôle du dégagement de chaleur est difficile à réaliser. La capacité des échangeurs thermiques destinés à refroidir le milieu réactionnel peut rapidement devenir insuffisante et un emballement dangereux peut dans certains cas se produire.

S. Urano et al ont récemment amélioré ce procédé pour la production d'isocyanate d'acryloyle et d'isocyanate de méthacryloyle (brevet US n° 4 925 982). Si ces isocyanates sont effectivement produits avec un rendement acceptable, ils ne sont malheureusement pas purs. En effet les isocyanates de propionyle β-halogénés correspondants sont formés en même temps. Il est par conséquent nécessaire d'effectuer une séparation pénible de ces deux types d'isocyanates.

S. Urano et al ont alors proposé de préparer les isocyanates d'alkénoyle par décomposition d'un halogénohydrate d'oxazolinedione formé en ajoutant progressivement l'amide à la solution de chlorure d'oxalyle (brevet US n° 4 769 485). Cependant cette formation est aussi très fortement exothermique et difficile à contrôler. Le procédé peut être également dangereux car toute la quantité de chlorure d'oxalyle nécessaire est présente en même temps dans le réacteur. On dilue par conséquent généralement le chlorure d'oxalyle dans une grande quantité de solvant. Il en résulte des problèmes de solubilisation et d'agitation. La productivité du procédé est alors très faible.

La présente invention a pour objet un procédé de préparation d'isocyanates d'acyle qui ne présente pas les inconvénients des procédés antérieurs et qui permet d'obtenir de nombreux isocyanates d'acyle de pureté élevée, sans danger et avec un très bon rendement.

Selon la présente invention, le procédé de préparation des isocyanates d'acyle de formule $Z(CONCO)_n$ dans laquelle Z représente un radical mono ou divalent,

- aliphatique en $C_1$ à $C_{20}$, linéaire ou ramifié, saturé ou non, substitué ou non,
- cycloaliphatique en $C_5$ à $C_7$, saturé ou non, substitué on non,
- araliphatique en $C_7$ à $c_{20}$, linéaire ou ramifié, saturé ou non, substitué ou non,
- phényle, phénylène, naphtyle ou naphtylène, substitué ou non,
- hétérocyclique à 5 ou 6 sommets, comportant un ou plusieurs hétéroatomes choisi(s) parmi l'oxygène, l'azote et le soufre, saturé ou non, substitué ou non,

ou Z représente un groupe RO-, -O-R-O- ou -R-O- dans lequel R représente un radical mono ou divalent,

- aliphatique en $C_1$ à $C_8$, linéaire ou ramifié, saturé ou non, substitué ou non
- cycloaliphatique en $C_5$ à $C_7$, saturé ou non, substitué ou non,
- araliphatique en $C_7$ à $C_{11}$, linéaire ou ramifié, saturé ou non, substitué ou non,
- phényle, phénylène, naphtyle ou naphtylène, substitué ou non,

et n représente le nombre 1 ou 2,
consiste à faire réagir le chlorure d'oxalyle avec un sel de carboxamides et/ou de carbamates, de formule $Z(CONH_2)_n$, $y(H_mX)$, dans laquelle Z et n ont les signications précédentes, m représente le nombre 1 ou 2, y représente la valeur $\frac{n}{m}$ et X représente un atome d'halogène, le groupe $SO_4$ ou $HSO_4$, à une température comprise entre à 15° et 30°C ou égale à ces valeurs, sans refroidir le milieu réactionnel et sans ajouter d'acide halogénohydrique, puis à chauffer le milieu réactionnel à une température comprise entre 30°C et 150°C.

Le ou les substituants de Z peuvent en particulier être choisis dans le groupe constitué par les atomes d'halogène et les radicaux hydrocarbyloxy et halogénohydrocarbyloxy en $C_1$ à $C_{20}$, par exemple tels que méthoxy, phényloxy, chlorophényloxy.

Lorsque Z représente un groupe comportant un ou plusieurs radicaux aromatiques ou hétéroaromatiques, le ou les substituants de ces radicaux peuvent également être choisis parmi les radicaux hydrocarbyle et halogénohydrocarbyle en $C_1$ à $C_{20}$, par exemple tels que méthyle, éthyle, trifluorométhyle, phényle, chlorophényle, fluorophényle, et le groupe nitro.

Les liaisons insaturées des radicaux aliphatiques ou araliphatiques peuvent être de type éthylénique ou

acétylénique. Dans les radicaux cycloaliphatiques ou hétérocycliques non aromatiques elles sont de type éthylénique.

Les atomes d'halogène sont de préférence choisis parmi les atomes de chlore, de brome et de fluor.

Les hétéroatomes des radicaux hétérocycliques sont de préférence choisis parmi les atomes d'oxygène et d'azote.

X peut représenter un atome d'halogène tel que de chlore, de brome et d'iode. Pour des raisons économiques X représente de préférence un atome de chlore.

La réaction peut être représentée par le schéma général suivant

$$Z(CONH_2)_n,\ y(H_mX) + nClCOCOCl \rightarrow$$

$$Z(CONCO)_n + 2nHCl + nCO + yH_mX$$

Les sels de carboxamides et/ou de carbamates utilisés comme matière de départ dans le cadre de la présente invention sont disponibles dans le commerce ou peuvent se préparer facilement en particulier par réaction des amides et des carbamates avec l'acide sulfurique ou les acides halogénohydriques telles que l'acide chlorhydrique, l'acide bromhydrique ou l'acide iodhydrique.

Tous les carboxamides et les carbamates, non substitués sur l'azote, qui peuvent former un sel avec les acides précédemment cités peuvent être mis à réagir avec le chlorure d'oxalyle.

Pour des raisons économiques, on préfère généralement utiliser les chlorhydrates d'amides et les chlorhydrates de carbamates. Ces chlorhydrates peuvent par exemple être préparés en faisant passer un courant d'acide chlorhydrique gazeux, de préférence anhydre, dans une suspension ou une solution du carboxamide ou du carbamate dans un milieu solvant organique inerte. Un équivalent au moins d'acide chlorhydrique par équivalent de fonction amide ou carbamate à salifier doit être introduit. On peut réaliser la préparation du sel dans un milieu solvant utilisable pour la réaction avec le chlorure d'oxalyle ce qui évite l'opération de séparation du sel et du solvant et ensuite l'introduction d'un nouveau solvant. Dans ce cas on vérifie que le carboxamide ou le carbamate a été totalement transformé en sel avant d'effectuer la réaction avec le chlorure d'oxalyle.

Parmi les isocyanates d'acyle pouvant être préparés de façon avantageuse par le procédé selon l'invention on peut citer :

- les isocyanates d'acétyle (Z = méthyle), de propionyle (Z = éthyle), de butyryle (Z = propyle), d'isobutyryle (Z = isopropyle), de pivaloyle (Z = t-butyle), de stéaroyle (Z = $CH_3(CH_2)_{16}$), de trichloroacétyle (Z = $-CCl_3$);
- les isocyanates de cyclopentanecarbonyle (Z = cyclopentyle), de cyclohexanecarbonyle (Z = cyclohexyle);
- l'isocyanate de phénylacétyle (Z = benzyle);
- les isocyanates d'acryloyle (Z = vinyle), de méthacryloyle (Z = isopropényle), de cinnamoyle (Z = $C_6H_5$-CH=CH-),
- les isocyanates de benzoyle (Z = phényle), de p-méthoxybenzoyle (Z = p-méthoxyphényle), de 2,6-difluorobenzoyle (Z = 2,6-difluorophényle), de 2,6-dichloro-3-nitrobenzoyle (Z = 2,6-dichloro-3-nitrophényle), de 1- ou 2-naphtoyle (Z = napthyle), de 5-chloro-2-nitrobenzoyle (Z = 5-chloro-2-nitrophényle),
- l'isocyanate de 5-nitrofuroyle (z = 5-nitrofuryle);
- les isocyanates de succinyle (z = $-CH_2CH_2-$, n = 2), d'adipoyle (z = $-(CH_2)_4-$, n = 2), de téréphtaloyle (z = p $-C_6H_4-$, n = n = 2), 2); de fumaryle (z = -CH=CH-, n=2)
- les isocyanates de méthoxycarbonyle (z = $CH_3O-$), d'éthoxycarbonyle (z = $C_2H_5O-$), de t-butoxycarbonyle (z = t-butyl-O-), de 2,2,2-trichloroéthoxycarbonyle (z = $Cl_3CCH_2O-$), de benzyloxycarbonyle (z = $C_6H_5-CH_2O-$), de phénoxycarbonyle (z = $C_6H_5-O-$),
- le diisocyanate de butylène bis 1,4-(oxycarbonyle).

Le procédé selon l'invention peut être mis en oeuvre sans solvant, mais de préférence on utilise un solvant ou un mélange de solvants organiques aprotiques et inertes vis à vis des composés, de préférence anhydres. Parmi les solvants convenant bien, on peut citer : les hydrocarbures aliphatiques halogénés ou non, tels que l'hexane, le dichlorométhane et le 1,2-dichloroéthane, les cycloalcanes tels que le cyclohexane et le méthylcyclohexane, les hydrocarbures aromatiques tels que le toluène, les xylènes, le chlorobenzène, l'o-dichlorobenzène. Pour la préparation des isocyanates d'alkénoyle, en particulier de l'isocyanate de méthacryloyle, un mélange d'o-dichlorobenzène et d'un alcane léger tel que le n-hexane, le n-heptane ou le cyclohexane convient bien.

De préférence le chlorure d'oxalyle est introduit dans le milieu contenant le sel de carboxamide ou de carbamate et en particulier de façon progressive. De cette façon il est consommé au fur à mesure et les risques sont ainsi limités. De plus comme il est liquide son addition est facile.

Une quantité environ stoechiométrique de chlorure d'oxalyle par fonction sel d'amide ou sel de carbamate à transformer est nécessaire, de préférence on ajoute de 1 à 1,2 équivalent de chlorure d'oxalyle par fonction sel d'amide ou de carbamate, c'est-à-dire un excès compris entre 0 et 20 %.

Contrairement aux procédés antérieurs dans lesquels la réaction du chlorure d'oxalyle avec un amide se produit avec un échauffement important du milieu réactionnel, la réaction du chlorure d'oxalyle avec le sel de l'amide ou du carbamate selon le procédé de l'invention, est une réaction globalement athermique qui ne nécessite aucun transfert de chaleur et en particulier

aucun refroidissement. Elle peut par conséquent s'effectuer à la température ambiante, en général comprise entre 15° et 30°C ou égale à ces valeurs lorsque les composés sont stables à cette température dans les conditions réactionnelles, et aucun risque d'emballement n'est à craindre.

Une fois l'addition du chlorure d'oxalyle terminée, on laisse si nécessaire le mélange réactionnel sous agitation afin que tout le sel soit bien transformé puis l'on chauffe le milieu réactionnel à une température comprise entre 30°C et 150°C, de préférence entre 35°C et 130°C.

Le milieu solvant est généralement choisi de façon que son point d'ébullition soit suffisamment élevé pour permettre d'atteindre ces températures. Si le milieu solvant n'a pas un point d'ébullition suffisamment élevé, on peut, avant de chauffer, changer de milieu solvant ou on peut ajouter un autre solvant convenable.

L'isocyanate d'acyle obtenu est séparé du milieu réactionnel, selon les techniques habituelles, par exemple par filtration ou par distillation à la pression atmosphérique ou sous pression réduite.

Le rendement de certains isocyanates, en particulier celui de l'isocyanate de méthacryloyle peut être amélioré si, avant d'effectuer le chauffage du milieu réactionnel, on attend la précipitation du composé intermédiaire qui se forme généralement au cours de la réaction. Si cette précipitation ne s'effectue pas spontanément on peut l'amorcer par tout moyen connu, par exemple en ajoutant dans le milieu réactionnel une petite quantité du produit intermédiaire obtenu dans une précédente opération et/ou par un léger chauffage jusqu'à une température pouvant atteindre 30°C.

Le procédé selon l'invention peut être également réalisé dans certains cas, en deux étapes bien distinctes. On effectue alors la réaction du chlorure d'oxalyle et du sel de carboxamide ou de carbamate comme précédemment décrit et on sépare du milieu réactionnel, le ou les produits obtenus, selon les techniques habituelles. Ceux-ci peuvent être conservés le temps souhaité puis transformés par chauffage dans les conditions précédemment mentionnées.

Le procédé selon l'invention, permet la préparation industrielle, économique et sans danger de très nombreux isocyanates dérivés de carboxamides et de carbamates. En particulier les isocyanates d'acyle dérivés d'amides aliphatiques qui n'étaient obtenus qu'avec de très mauvais rendements selon le procédé de A.J. Speziale et al sont grâce à ce nouveau procédé obtenus avec d'excellents rendements.

Les isocyanates d'acyle sont très utiles pour la préparation de nombreux produits du domaine de la pharmacie, de l'agrochimie et des polymères. On peut citer par exemple l'isocyanate d'acétyle utilisé comme intermédiaire clé dans la préparation de triazolinones (J. Heterocyl. Chem 1990, 27(7), 2017). L'isocyanate de trichloroacétyle est très employé pour former des fonctions carbamates au cours de la synthèse d'antibiotiques en particulier de la "céfuroxime" (CA 94 84147 e). D'autres antibiotiques sont fabriqués au moyen des isocyanates de t-butoxycarbonyle, de 2,2,2-trichloroéthoxycarbonyle et de benzyloxycarbonyle.

L'isocyanate de méthacryloyle très réactif permet par copolymérisation d'obtenir des matériaux utilisables pour des revêtements réticulables, pour des adhésifs dentaires ou pour des élastomères acryliques. De nouveaux médicaments tels que les inhibiteurs de lipoxygénase sont préparés à partir d'isocyanate de benzoyle. Les isocyanates de benzoyle substitués tels que l'isocyanate de 2,6-difluorobenzoyle sont très utilisés pour fabriquer de nombreuses benzoylurées insecticides. L'isocyanate de benzyloxycarbonyle permet la synthèse directe d'amines protégées (Synthesis, Déc. 1988, p. 992).

Les exemples qui suivent sont destinés à illustrer la présente invention sans toutefois la limiter.

Toutes les réactions sont effectuées en atmosphère inerte à partir de matières premières du commerce.

L'acide chlorhydrique utilisé est anhydre.

Exemple 1 : Préparation de l'isocyanate de benzoyle

On introduit 36,3 g (0,3 mol) de benzamide et 200 g de dichlorométhane dans un réacteur de 250 ml. On fait passer ensuite dans le milieu, sous agitation, 13,8 g (0,378 mol) d'acide chlorhydrique gazeux en 44 minutes, bulle à bulle. Durant l'addition d'HCl, la température passe de 21,3°C à 38°C.

Le chlorhydrate de benzamide précipite sous forme d'un solide blanc. Le spectre RMN $^1H$ confirme que la benzamide a disparu et que l'on a obtenu son chlorhydrate :

| | |
|---|---|
| ($CDCl_3$, ppm) : | 7,53 (2H arom., t) |
| | 7,58 (1H arom., t) |
| | 8,10 (2H arom., t) |
| | 11,0-8,5 (H de NH) |

On ajoute ensuite, goutte à goutte, à la suspension agitée de chlorhydrate, 42 g (0,33 mol) de chlorure d'oxalyle, pendant 39 minutes, et cela sans refroidir. La température du milieu passe graduellement de 21,3°C à 16°C et on observe un vigoureux dégagement d'HCl.

On laisse sous agitation pendant 30 min, puis on porte à reflux (température du milieu : 38° à 46°C; température du bain d'huile : 60°C) jusqu'à disparition du solide en suspension.

On arrête le chauffage après 3 heures et on refroidit le milieu.

Le taux de transformation de la benzamide en isocyanate de benzoyle obtenu déterminé par RMN $^1H$ est de 94 %

($CDCl_3$ ; ppm) :protons aromatiques à 7,33-7,47 ; 7,51-7,64 et 7,91-8,02.

Exemple 2 : Préparation de l'isocyanate de benzoyle à partir de la benzamide (Exemple comparatif)

Cet exemple ne fait pas partie de l'invention et a été effectué dans le seul but de montrer les avantages du procédé selon l'invention.

On utilise le même appareillage que celui utilisé à l'exemple 1.

A une solution-suspension agitée de 36,3 g (0,3 mol) de benzamide dans 200 g de dichlorométhane, on ajoute, goutte à goutte, en 38 minutes, 42 g (0,33 mol) de chlorure d'oxalyle. La température s'élève de 19,6°C à 30,5°C dès l'addition d'un tiers du chlorure d'oxalyle.

Une fois l'addition du chlorure d'oxalyle terminée on opère comme à l'exemple 1. Cependant, dans ce cas, on constate que le solide blanc formé ne disparait pas complètement.

Le taux de transformation en isocyanate de benzoyle mesuré comme précédemment est de 88,6 %.

Exemple 3 : Préparation de l'isocyanate de méthacryloyle

On introduit 102 g (1,2 mol) de méthacrylamide, 240 g de d'o-dichlorobenzène et 50 g d'hexane dans un réacteur de 1000 ml.

On agite la suspension à température ambiante et on introduit de l'acide chlorhydrique gazeux sans refroidir le milieu. Après introduction de 1,2 à 1,3 éq. (équivalent) de HCl par rapport à la méthacrylamide, on refroidit le milieu à 18°C. Le chlorhydrate de la méthacrylamide obtenu est caractérisé par son spectre RMN $^1H$ :

| | |
|---|---|
| ($CDCl_3$ ; ppm) : | 2,15 ($CH_3$) |
| | 6,0 (H, éthylénique) |
| | 6,5 (H, éthylénique) |

On ajoute ensuite goutte à goutte, sous agitation, en 45 min, 167 g (1,1 éq.) de chlorure d'oxalyle. On constate une absence totale de dégagement de chaleur.

On fait précipiter le produit formé, si nécessaire par addition d'une petite quantité de ce même produit issu d'une opération précédente et/ou par un léger chauffage jusqu'à une température de 30°C.

Lorsque la précipitation est terminée, on introduit, en une heure, 310 g d'hexane.

On chauffe alors le milieu réactionnel à 72°-74°C jusqu'à ce qu'un changement d'aspect accompagné d'une élévation de température (environ + 5°C) soit noté (environ 2 heures).

On poursuit le chauffage encore 20 min, puis on refroidit le milieu jusqu'à la température ambiante.

Le taux de transformation de la méthacrylamide en isocyanate de méthacryloyle déterminé par RMN $^1H$ est de 90-100 %.

Exemple 4 : Préparation de l'isocyanate de triméthyl acétyle

On introduit 30 g (0,29 mol) de triméthylacétamide et 200 g de d'o-dichlorobenzène dans un réacteur de 500 ml. On fait passer ensuite 14 g (0,38 mol) d'acide chlorhydrique gazeux en 35 min, bulle à bulle, dans le milieu agité. Durant cette phase, la température monte de 20,0°C à 31,5°C. On constate également la disparition du solide en suspension qui était du triméthylacétamide (celui ci n'étant pas complètement soluble dans le milieu). On obtient ainsi une solution limpide de chlorhydrate de triméthylacétamide comme déterminé par le spectre RMN $^1H$.

On ajoute ensuite goutte à goutte, 41 g (0,32 mol) de chlorure d'oxalyle à la solution précédente à 19,6°C. Pendant l'addition qui dure 35 min, la température n'évolue pratiquement pas et reste comprise entre 19° et 21°C.

On agite 10 min à 30°C, puis on chauffe pendant une heure à l'aide d'un bain d'huile à 80°C, la température du milieu évolue de 65° à 80°C. La formation du produit attendu est suivi par spectrométrie infra-rouge (bande NCO à 2 220 $cm^{-1}$).

On isole ensuite l'isocyanate de triméthylacétyle par distillation sous pression réduite (Point d'ébullition (Eb) : 62°C/110 mmHg)

| | |
|---|---|
| Rendement : | 75 % |
| RMN $^1H$ ($CDCl_3$) : | 1,15 ppm (s, t-butyle). |

Exemple 5 : Préparation de l'isocyanate de méthoxycarbonyle

$CH_3$-O-CO-NCO

On introduit 40 g (0,53 mol) de carbamate de méthyle et 200 g d'o-dichlorobenzène dans un réacteur de 500 ml. On fait passer, comme indiqué à l'exemple 4, 28 g (0,75 mol) d'acide chlorhydrique gazeux pendant 90 min. La température passe de 20°C à 29°C. On obtient ainsi le chlorhydrate du carbamate sous forme d'une émulsion dans le solvant comme déterminé par le spectre RMN $^1H$ ($CDCl_3$) : protons groupe -$NH_2$ à 5,04 ppm.

On ajoute ensuite, goutte à goutte, 74,5 g (0,59 mol) de chlorure d'oxalyle à l'émulsion précédente pendant 50 min. Durant cette opération la température évolue de 20°C à 22°C.

On continue à agiter 30 min. à 26°C, puis on chauffe le milieu de 70° à 130°C pendant 4 h 30 min..

On isole ensuite l'isocyanate de méthoxycarbonyle par distillation sous pression ordinaire (Eb : 68°-70°C)

| | |
|---|---|
| Rendement : | 55 % |
| RMN $^1H$ ($CDCl_3$) : | 3,82 ppm (s, méthyle). |

Exemple 6 : Préparation de l'isocyanate de phénylacétyle
$C_6H_5$-$CH_2$-CO-NCO

On introduit 20 g (0,148 mol) de phénylacétamide et 150 g de 1,2-dichloroéthane dans un réacteur de 250 ml. On fait passer, comme indiqué à l'exemple 4, 12 g (0,32 mol) d'acide chlorhydrique gazeux en 60 min. La température passe de 21,5°C à 42,2°C. On obtient ainsi une suspension du chlorhydrate, solide blanc, dont la structure est confirmée par RMN $^1$H ($CDCl_3$) :

protons $CH_2$ : 3,75 ppm
protons $NH_2$ : 3,8-3,9 (large).

On ajoute, goutte à goutte, sous agitation à la suspension précédente, 22 g (0,173 mol) de chlorure d'oxalyle pendant 35 min. Durant cette opération la température du milieu reste voisine de 19°C et la couleur du solide en suspension passe du blanc au jaune.

On continue à agiter 30 min, à 30°C. On recueille le produit intermédiaire par filtration puis on le met en suspension dans 150 g d'o-dichlorobenzène sous agitation. On porte la suspension à une température comprise entre 136° et 146°C pendant 8 heures.

On élimine ensuite le solvant par évaporation sous vide et on purifie l''isocyanate de phénylacétyle par distillation sous pression réduite (Eb : 78°C/3-5 mmHg)

Rendement : 47 %
RMN $^1$H ($CDCl_3$) : protons aromatiques : 7,37-7,04 ppm (m), protons -$CH_2$- : 3,66 ppm.

Exemple 7 : Préparation de l'isocyanate de phénylacétyle à partir de la phénylacétamide (Exemple comparatif)

Cet exemple ne fait pas partie de l'invention.

On introduit 20 g (0,148 mol) de phénylacétamide et 150 g de 1,2-dichloroéthane dans un réacteur de 250 ml. On introduit ensuite, sous agitation en 35 min, 22 g (0,173 mol) de chlorure d'oxalyle dans la suspension. La température évolue de 22°C à 33,7°C.

On opère ensuite exactement comme à l'exemple 6 à partir de la séparation du produit intermédiaire.

Après distillation sous pression réduite dans les mêmes conditions, l'isocyanate de phénylacétyle est obtenu avec un rendement de 28 % seulement.

Exemple 8 : Préparation de l'isocyanate de phénoxy carbonyle
$C_6H_5$-O-CO-NCO.

On place 41,14 g (0,3 mol) de carbamate de phényle et 200 g de 1,2-dichloroéthane dans un réacteur de 300 ml. On fait passer, bulle à bulle, 15,3 g (0,42 mol) d'HCl gazeux dans la suspension-solution pendant 100 min. La température s'élève de 22°C à 26°C. Le chlorhydrate de carbamate de phényle, solide blanc, précipite.

Le spectre RMN $^1$H effectué confirme cette forme.

($CDCl_3$, ppm) : 7,12-7,15 (2H arom.,) 7,15-7,22 (1H arom.,)
7,35-7,39 (2H arom.,)
5,16 (H de NH)

On ajoute, goutte à goutte, 41,91 g (0,33 mol) de chlorure d'oxalyle, à la suspension de chlorhydrate de carbamate de phényle obtenue, pendant 40 min. sans refroidir.

La température de la réaction est comprise entre 20°C à 22°C. On continue à agiter à 22°C pendant 30 min.

On chauffe ensuite le milieu réactionnel de 45°C à 93°C pendant 7 heures.

On élimine ensuite le solvant par évaporation sous pression réduite et on purifie l'isocyanate de phénoxycarbonyle par distillation sous pression réduite (Eb. : 125°C/5 mmHg)

Rendement : 57,6 %
RMN $^1$H ($CDCl_3$) : 7,26-7,35 ppm (phényle)

Exemple 9 : Préparation de l'isocyanate de 5-chloro-2-nitrobenzoyle

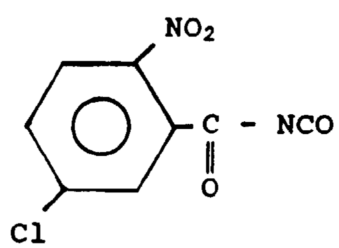

On place 39,965 g (0,199 mol) de 5-chloro-2-nitrobenzamide et 200 g de 1,2-dichloroéthane dans un réacteur de 500 ml. On fait passer, bulle à bulle, 11,0 g (0,31 mol) de HCl gazeux dans la suspension-solution pendant 80 min.. La température s'élève de 25°C à 35°C.

Le chlorhydrate de 5-chloro-2-nitrobenzamide, solide blanc, précipite.

Sa structure est confirmée par le spectre RMN $^1$H

(DMSO, ppm) : 7,708-7,738 (H arom.,)
7,787-7,811 (H arom.,)
7,824-8,104 (H arom.,)
8,207 (H de NH)

On ajoute, goutte à goutte, 27,94 g (0,22 mol) de chlorure d'oxalyle, à la suspension de chlorhydrate pendant 40 min. sans refroidir. La température de la réaction s'élève de 18°C à 26°C. On continue ensuite à agiter à

25°C pendant 30 min. puis on chauffe à 65°C pendant 1 heure et demie.

Afin de caractériser l'isocyanate formé on le fait réagir avec de l'aniline, on obtient 23,8 g de N-(5-chloro-2-nitrobenzoyl) -N'-phénylurée.

Le rendement calculé à partir de la quantité obtenue de ce dérivé est de 38,4 %.

Exemple 10 : Préparation de l'isocyanate de 2,6-difluorobenzoyle

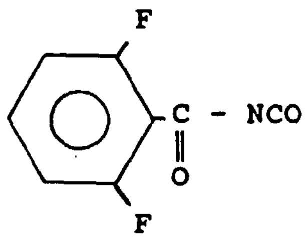

On introduit en 55 min, 11,8 g (0,32 mol) d'HCl gazeux dans une suspension-solution de 39,25 g (0,25 mol) de 2,6-difluorobenzamide dans 300 g de dichlorométhane. La température du milieu réactionnel s'élève de 21°C à 25°C. On contrôle la formation du chlorhydrate par analyse RMN $^1$H. Le signal d'un des protons du $NH_2$ du sel est déplacé légèrement (6,38 ppm) par rapport au signal du même proton du $NH_2$ de l'amide (6,33 ppm). Le signal du proton HCl est à 1,79 ppm (large s).

On laisse reposer la solution-suspension du chlorhydrate de 2,6-difluorobenzamide pendant 20 min puis on y ajoute 35 g (0,276 mol) de chlorure d'oxalyle à 23°C en 50 min. La température passe de 23°C à 19,6°C et on observe un dégagement d'HCl.

On laisse reposer le milieu réactionnel pendant 1 heure et demie puis on le chauffe à 40°C pendant 3 heures et demie. On isole ensuite 38 g d'isocyanate de 2,6-difluorobenzoyle par distillation sous pression réduite.

(Eb : 103-106°C/3 mm Hg)

Rendement : 83 %
IR : 2250 cm$^{-1}$ (NCO).

Exemple 11 : Préparation de l'isocyanate de cinnamoyle

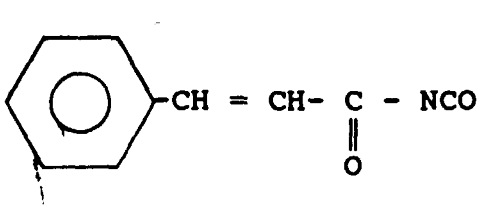

On introduit en 30 min 10,3 g (0,28 mol) d'HCl gazeux dans une suspension-solution de 25 g (0,17 mol) de cinnamide dans 150 g de 1,2-dichloroéthane. La température s'élevant de 21°C à 38°C, on refroidit le milieu. On contrôle la formation du chlorhydrate de la cinnamide par analyse RMN $^1$H :

($CDCl_3$, ppm) : 2,045 (large s, HCl)
5,65-6,1 (large, 2H, $NH_2$)
6,48 (d, 1H, vinyl J = 15,7 Hz)
7,36-7,44 et 7,48-7,58 (m, 5H, arom.)
7,67 (d, 1H, vinyl J = 15,7 Hz)

On laisse reposer le milieu réactionnel pendant 50 min, à la température ambiante puis on ajoute 24 g (0,189 mol) de chlorure d'oxalyle à 23°C en 30 min La température passe de 23°C à 22°C. On chauffe ensuite le milieu réactionnel à 80°-85°C pendant 1,6 heure puis on le refroidit. On distille le milieu sous pression réduite et on recueille 13,7 g d'isocyanate de cinnamoyle.

(Eb : 159-167°C/3 mm Hg)

Rendement : 46,6 %
IR : 2250 cm$^{-1}$ (NCO).

Exemple 12 : Préparation de l'isocyanate de cyclohexanoyle

On introduit en 50 min, 10,2 g (0,28 mol) d'HCl gazeux dans une suspension-solution de 25,4 g (0,20 mol) de cyclohexane carboxamide dans 100 g de 1,2-dichloroéthane. La température du milieu réactionnel s'élève de 23°C à 45°C et on obtient une solution limpide. La formation du chlorhydrate est contrôlée par analyse RMN $^1$H :

($CDCl_3$, ppm) :1,20-2,06 (m, 10H, $CH_2$)
2,67-2,82 (m, 1H, CH)
9,0-9,4 (large, 1H, $NH_2$)
9,65-10,1 (large, 1H, $NH_2$)
11,5-11,9 (large s, 1H, HCl)

On ajoute ensuite 27,9 g (0,22 mol) de chlorure d'oxalyle à 28°C en 40 min. La température passe de 28° à 18°C. Puis on chauffe le milieu réactionnel à 85-91°C pendant 4 heures et on le refroidit.

On isole 10,71 g d'isocyanate de cyclohexanoyle par distillation sous pression réduite.

(Eb : 88-90°C/3 mm Hg)

Rendement : 35 %
IR : 2238 cm$^{-1}$ (NCO)

Example 13 : Préparation de l'isocyanate de p-méthoxybenzoyle

On introduit en 25 min., 10,1 g (0,28 mol) d'HCl gazeux dans une suspension-solution de 30,2 g (0,2 mol) de p-méthoxybenzamide dans 200 g de dichlorométhane. La température du milieu réactionnel s'élève de 17°C à 31°C. On contrôle la formation du chlorhydrate par analyse RMN $^1$H :

($CDCl_3$, ppm) :3,78 ppm (s, 3H, $CH_3O$)
4,2-4,8 ppm (large, 1H, $NH_2HCl$)
6,98-7,88 (chacun d, 2H, arom. J = 8,65 Hz)

On laisse reposer le milieu réactionnel pendant 55 min., à la température ambiante, puis on ajoute 27,9 g (0,22 mol) de chlorure d'oxalyle en 50 min. à 22,6°C. La température du milieu passe de 24°C à 19°C. On chauffe ensuite le milieu réactionnel à 39-43°C pendant 3 heures et on le refroidit.

On isole 31,5 g d'isocyanate de p-méthoxybenzoyle par distillation sous pression réduite.

(Eb : 113°-116°C/2 mm Hg)

Rendement : 89 %
IR : 2245 $cm^{-1}$ (NCO).

Exemple 14 : Préparation de l'isocyanate de stéaroyle

On introduit en 2,9 heures, 14,1 g (0,38 mol) d'HCl gazeux dans une suspension-solution de 28,3 g (0,1 mol) de stéarylamide dans 200 g de dichlorométhane. Le milieu devenant progressivement solide, on ajoute 100 g de dichlorométhane. La température s'élève de 17°C à 21,7°C. On contrôle la formation du sel par analyse RMN $^1$H :

($CDCl_3$, ppm) :0,88 (t, 3H, $CH_3$, J = 6,8 Hz)
1,22-1,3 (large m, 30H, $CH_2$)
1,64 (large, 1H, $NH_2$)
2,09 (large, 1H, $NH_2$)
2,24 (m, 2H, $CH_2CO$)
5,6 (large, 1H, HCl)

On ajoute ensuite dans le milieu réactionnel, 13,9 g (0,11 mol) de chlorure d'oxalyle en 30 min à 20,7°C. La température du milieu passe de 20,6°C à 19°C.

On chauffe le milieu réactionnel à 39°-40°C pendant 4 heures et on n'observe pas la formation de l'isocyanate. Afin de pouvoir chauffer à une température plus élevée, on procède à un changement de solvant en remplaçant le dichlorométhane par le 1,2-dichloroéthane par voie azéotropique.

On chauffe ensuite à 84°-86°C pendant 6 heures et on refroidit le milieu réactionnel.

On isole 12,2 g d'isocyanate de stéaroyle par distillation sous pression réduite.

(Eb : 79°-82°C/4 mm Hg)

Rendement : 39 %
IR : 2245 $cm^{-1}$ (NCO).

Exemple 15 : Préparation de l'isocyanate de phénylacétyle

On forme une suspension de 12 g (0,07 mol) de chlorhydrate de phénylacétamide dans 80 g d'o-dichloro-benzène.

On ajoute, goutte à goutte, sous agitation, à cette suspension, 10 g (0,079 mol) de chlorure d'oxalyle pendant 30 min. On continue à agiter 30 min, à 30°C, puis on chauffe la suspension à une température comprise entre 136° et 146°C pendant 8 heures.

On élimine le solvant par évaporation sous pression réduite et on recueille 5,64 g d'isocyanate de phénylacétyle par distillation sous pression réduite.

(Eb : 78°/3-5 mm Hg)

Rendement : 50 %

Exemple 16 : Préparation de l'isocyanate de 2-méthylpropanoyle

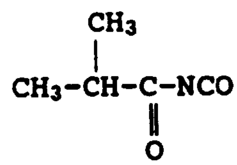

On introduit en 1,9 heure, 11,4 g (0,31 mol) d'HCl gazeux dans une suspension-solution de 17,4 g (0,2 mol) de 2-méthylpropionamide dans 200 g de 1,2-dichloroéthane. La température du milieu s'élève de 20,4°C à 41,9°C. On contrôle la formation du chlorhydrate par analyse RMN $^1$H :

($CDCl_3$, ppm) : 1,35 (d, 6H, $CH_3$)
2,9-3,1 (m, 1H, CH)
9,6-10,3 (large, $NH_2HCl$)

On laisse reposer le milieu réactionnel pendant 30 min., puis on ajoute 27,9 g (0,22 mol) de chlorure d'oxalyle, en 30 min. à 20,7°C. La température du milieu passe de 25,1° à 18°C. On chauffe ensuite le milieu réactionnel à 83°-86°C pendant 3,2 heures et on le refroidit.

L'isocyanate de 2-méthylpropanoyle obtenu est caractérisé par analyse RMN $^1$H et IR :

($CDCl_3$, ppm) : 1,14 (d, 3H, $CH_3$)
1,77 (d, 3H, $CH_3$) 2,32-2,68 (m, 1H, CH)
IR : 2245 $cm^{-1}$ (NCO).

On ajoute ensuite 18,4 g (0,4 mol) d'éthanol dans le milieu réactionnel pour transformer l'isocyanate en carbamate.

Le rendement déterminé à partir de la quantité de carbamate obtenue est de 57,6 %.

Exemple 17 : Préparation de l'isocyanate de 1-naphtoyle

On introduit 4,496 g (0,026 mol) de 1-naphtylamide et 50 g de toluène dans un réacteur de 100 ml. On fait passer 6,1 g (0,17 mol) d'HCl gazeux, bulle à bulle, dans la suspension-solution agitée de 1-naphtylamide, en 45 min. Pendant cette addition, la température du milieu s'élève de 24°C à 31°C. On obtient le chlorhydrate de 1-naphtylamide sous forme d'un solide blanc en suspension. Il est caractérisé par son spectre RMN $^1H$ :

(DMSO , ppm) : 7,2 (H, (H,naphtalénique).

On ajoute ensuite, goutte à goutte, 3,69 g (0,031 mol) de chlorure d'oxalyle à la suspension agitée de chlorhydrate de 1-naphtylamide, pendant 40 min., sans refroidir. La température du milieu passe de 27°C à 24°C. On continue à agiter pendant 30 min. à 24°C, puis on chauffe le milieu entre 75°C et 95°C pendant 3,5 heures.

On enlève les restes solides par filtration sous pression réduite. Le spectre IR de l'isocyanate de naphtoyle obtenu montre une absorption à 2240 $cm^{-1}$.

Le rendement déterminé à partir de la quantité de l'urée formée par réaction de l'isocyanate de naphtoyle avec l'aniline est de 98 %.

Exemple 18 : Préparation de l'isocyanate de succinyle

On introduit 15,1 g (0,31 mol) de succinamide et 300 g de 1,2-dichloroéthane dans un réacteur de 500 ml. On fait passer 14,6 g (0,41 mol) d'HCl gazeux, bulle à bulle, dans la suspension-solution agitée de succinamide en 20 min. Pendant cette addition, la température du milieu s'élève de 24°C 28°C. On obtient le chlorhydrate de succinamide sous forme d'un solide blanc en suspension. Il est caractérisé par son spectre RMN $^1H$ :

(DMSO, ppm) : 2,24 (H,méthylènique).

On ajoute ensuite, goutte à goutte, 66,3 g (0,52 mol) de chlorure d'oxalyle à la suspension agitée de chlorhydrate de succinamide, pendant 60 min., sans refroidir. La température du milieu passe de 26°C à 25°C. On continue à agiter pendant 60 min. à 25°C, puis on chauffe le milieu à 81°C pendant 9 heures.

On enlève ensuite les restes solides par filtration sous pression réduite. Le spectre IR de l'isocyanate de succinyle obtenu montre une absorption à 2240 $cm^{-1}$. Le rendement déterminé à partir de l'urée formée par réaction de l'isocyanate de succinyle avec l'aniline est de 77,3 %.

Exemple 19 : Préparation de l'isocyanate de téréphtaloyle

On introduit 32,38 g (0,2 mol) de téréphtalamide et 200 g de 1,2-dichloroéthane dans un réacteur de 500 ml. On fait passer, bulle à bulle, 16,7 g (0,47 mol) d'HCl gazeux dans la suspension-solution agitée de téréphtalamide en 40 min. Pendant cette addition, la température du milieu s'élève de 20°C 25°C. On obtient le chlorhydrate de téréphtalamide sous forme d'un solide blanc en suspension. Il est caractérisé par son spectre RMN $^1H$ ($CDCl_3$, ppm) : 3,37 (H de NH)

On ajoute ensuite, goutte à goutte, 101,6 g (0,8 mol) de chlorure d'oxalyle à la suspension agitée de chlorhydrate de téréphtalamide à 22°C, pendant 30 min., sans refroidir. On continue à agiter pendant 30 min. à 25°C, puis on chauffe le milieu à 68°C pendant 135 min..

On enlève les restes solides par filtration sous pression réduite. Le spectre IR de l'isocyanate de téréphtaloyle obtenu montre une absorption à 2230 $cm^{-1}$. Le rendement déterminé à partir de l'urée formée par réaction de l'isocyanate de téréphtaloyle avec l'aniline est de 78 %.

Exemple 20: Préparation de l'isocyanate d'adipoyle

On introduit 8,64 g (0,06 mol) d'adipamide et 100 g de 1,2-dichloroéthane dans un réacteur de 300 ml. On fait ensuite passer 11,9 g (0,32 mol) d'HCl gazeux, bulle à bulle, dans la suspension-solution agitée d'adipamide, en 50 min. Pendant cette addition, la température du milieu s'élève de 28°C à 31°C. On obtient le chlorhydrate d'adipamide sous forme d'un solide blanc en suspension. Il est caractérisé par son spectre RMN $^1H$ : (DMSO, ppm) : 1,45; 2,03 (H,méthylénique), 5,51 (H de NH).

On ajoute ensuite, goutte à goutte, 68,5 g (0,54 mol) de chlorure d'oxalyle à la suspension-solution agitée de chlorhydrate d'adipamide, pendant 20 min., sans refroidir. La température du milieu passe de 33°C à 30°C. On continue à agiter le milieu pendant 30 min. à 30°C, puis on le chauffe à 80°C pendant 1,5 heure.

Le spectre IR montre l'absorption de l'isocyanate d'adipoyle à 2250 $cm^{-1}$ (NCO).

Exemple 21: Préparation de l'isocyanate d'adipoyle (exemple comparatif)

Cet exemple ne fait pas partie de l'invention.

On introduit 8,64 g (0,06 mol) d'adipamide et 100 g de 1,2-dichloroéthane dans un réacteur de 300 ml. On ajoute 68,5 g (0,54 mol) de chlorure d'oxalyle à la suspension-solution agitée d'adipamide. Afin d'éviter un échauffement important du milieu réactionnel cette addition est faite goutte à goutte pendant 180 min.. La température du milieu s'élève de 28°C à 31°C. On continue à agiter le milieu pendant 30 min. à 31°C. puis on le chauffe à 80°C. Le spectre IR montre l'absorption de l'isocyanate d'adipoyle à 2250 $cm^{-1}$ (NCO) seulement après 12 heures de chauffage.

Exemple 22: Préparation de l'isocyanate de fumaroyle

On introduit 10,27 g (0,09 mol) de fumaramide et 400 g de 1,2-dichloroéthane dans un réacteur de 500 ml. On fait ensuite passer 10,44 g (0,28 mol) d'HCl gazeux, bulle à bulle, dans la suspension-solution agitée de fumaramide, en 25 min. Pendant cette addition, la température du milieu s'élève de 25°C à 32°C. On obtient le chlorhydrate de fumaramide sous forme d'un solide blanc en suspension. Il est caractérisé par son spectre RMN $^1$H : (DMSO, ppm) : 6,77 (H, méthylénique).

On ajoute ensuite, goutte à goutte, 111,8 g (0,90 mol) de chlorure d'oxalyle à la suspension-solution agitée de chlorhydrate de fumaramide, pendant 20 min., sans refroidir. La température du milieu reste à 24°C. On continue à agiter le milieu pendant 60 min. à 24°C, puis on le chauffe à 83°C pendant 3,5 heures.

On enlève ensuite les restes solides par filtration sous pression réduite. Le spectre IR de l'isocyanate de fumaroyle obtenu montre une absorption à 2240 $cm^{-1}$. Le rendement calculé à partir de la quantité de l'urée formée par réaction de l'isocyanate de fumaroyle avec l'aniline est de 61,5 %.

Exemple 23 : Préparation de l'isocyanate de tertiobutyloxycarbonyle

On introduit 4,0 g (0,034 mol) de carbamate de tertiobutyle et 80 g de 1,2-dichloroéthane dans un réacteur de 100 ml. Le carbamate de tertiobutyle étant instable à la température ambiante en présence d'acide chlorhydrique, on refroidit à 0°C la suspension-solution de carbamate de t-butyle puis on introduit dans cette suspension-solution agitée, 1,9 g (0,051 mol) d'HCl gazeux, bulle à bulle, pendant 90 minutes. La température s'élève de 0° à 3°C. On obtient le chlorhydrate du carbamate de t-butyle sous forme d'un solide blanc en suspension.

On ajoute ensuite, goutte à goutte, 8,3 g (0,063 mol) de chlorure d'oxalyle dans la solution-suspension agitée du chlorhydrate de carbamate de t-butyle à 0°C, pendant 20 minutes. La température du milieu s'élève de 0° à 2°C. On continue à agiter le milieu à 0°C pendant 90 minutes, puis on le chauffe à 80°C pendant 11 heures.

On enlève ensuite les restes solides par filtration sous pression réduite. Le spectre IR de l'isocyanate de tertiobutyloxycarbonyle obtenu montre une absorption à 2250 $cm^{-1}$. Le rendement déterminé à partir de l'urée formée par réaction de l'isocyanate de t-butyloxycarbonyle avec l'aniline est de 47,8%.

Exemple 24 : Préparation de l'isocyanate de n-butyryle

On introduit 25,8 g (0,30 mol) de n-butyramide et 200 g de 1,2-dichloroéthane dans un réacteur de 500 ml. On fait ensuite passer 13,6 g (0,36 mol) d'HCl gazeux, bulle à bulle, dans la suspension-solution agitée de n-butyramide, en 40 min. Pendant cette addition, la température du milieu s'élève de 20°C à 23°C. On obtient le chlorhydrate de n-butyramide sous forme d'un solide blanc en suspension. Il est caractérisé par son spectre RMN $^1$H.

On ajoute ensuite, goutte à goutte, 42,0 g (0,33 mol) de chlorure d'oxalyle à la suspension-solution agitée de chlorhydrate de butyramide, pendant 60 min., sans refroidir. La température du milieu passe de 23°C à 20°C. On continue à agiter le milieu pendant 60 min. à 20°C, puis on le chauffe à 120°C pendant 3 heures.

On enlève ensuite les restes solides par filtration sous pression réduite. Puis on isole l'isocyanate de n-butyryle par distillation sous pression réduite.

| | |
|---|---|
| Eb : | 64°C/40 mm Hg |
| Rendement : | 86 % |

**Revendications**

**1.** Procédé de préparation d'un isocyanate d'acyle de formule $Z(CONCO)_n$ dans laquelle Z représente un radical mono ou divalent,

- aliphatique en $C_1$ à $C_{20}$, linéaire ou ramifié, saturé ou non, substitué ou non,
- cycloaliphatique en $C_5$ à $C_7$, saturé ou non, substitué on non,
- araliphatique en $C_7$ à $c_{20}$, linéaire ou ramifié, saturé ou non, substitué ou non,
- phényle, phénylène, naphtyle ou naphtylène, substitué ou non,
- hétérocyclique à 5 ou 6 sommets, comportant un ou plusieurs hétéroatomes choisi(s) parmi l'oxygène, l'azote et le soufre, saturé ou non, substitué ou non,

ou Z représente un groupe RO-, -O-R-O- ou -R-O- dans lequel R représente un radical mono ou divalent,

- aliphatique en $C_1$ à $C_8$, linéaire ou ramifié, saturé ou non, substitué ou non
- cycloaliphatique en $C_5$ à $C_7$, saturé ou non, substitué ou non,
- araliphatique en $C_7$ à $C_{11}$, linéaire ou ramifié, saturé ou non, substitué ou non,
- phényle, phénylène, naphtyle ou naphtylène, substitué ou non,

et n représente le nombre 1 ou 2,
caractérisé en ce que l'on fait réagir le chlorure d'oxalyle avec un sel de formule $Z(CONH_2)_n$, y $(H_mX)$, dans laquelle Z et n ont les significations précédentes, m représente le nombre 1 ou 2, y représente la valeur $\frac{n}{m}$ et X représente un atome d'halogène, le groupe $SO_4$ ou $HSO_4$, à une température comprise entre à 15° et 30°C ou égale à ces valeurs, sans refroidir le milieu réactionnel et sans

ajouter d'acide halogénohydrique, puis en ce que l'on chauffe le milieu réactionnel à une température comprise entre 30°C et 150°C.

**2.** Procédé selon la revendication 1, caractérisé en ce que les substituants de Z sont choisis parmi les atomes d'halogène et les radicaux hydrocarbyloxy et halogénohydrocarbyloxy en $C_1$ à $C_{20}$ et de plus parmi les radicaux hydrocarbyle et halogénohydrocarbyle en $C_1$ à $C_{20}$ et le groupe nitro lorsqu'ils sont substitués sur un cycle aromatique ou hétéroaromatique.

**3.** Procédé selon la revendication 1 ou 2, caractérisé en ce que X représente un atome de chlore et m est égal à 1.

**4.** Procédé selon l'une quelconque des revendications précédentes, caractérisé en ce qu'il est mis en oeuvre dans un milieu solvant organique inerte et aprotique.

**5.** Procédé selon la revendication 4, caractérisé en ce que le milieu solvant est choisi dans le groupe constitué par les hydrocarbures aliphatiques halogénés ou non, les cycloalcanes, les hydrocarbures aromatiques, et leurs mélanges.

**6.** Procédé selon l'une quelconque des revendications précédentes, caractérisé en ce que Z représente un radical insaturé, substitué ou non, aliphatique, cycloaliphatique ou araliphatique.

**7.** Procédé selon la revendication 6, caractérisé en ce que Z représente le radical $CH_2 = CH_2$- ou $CH_2$ =

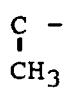

**8.** Procédé selon la revendication 7, caractérisé en ce qu'on utilise comme milieu solvant un mélange d'o-dichlorobenzène et d'un alcane léger.

**9.** Procédé selon l'une quelconque des revendications précédentes, caractérisé en ce qu'on utilise de 1 à 1,2 équivalent de chlorure d'oxalyle par fonction sel à faire réagir.

**10.** Procédé selon l'une quelconque des revendications précédentes, caractérisé en ce qu'on ajoute le chlorure d'oxalyle au sel.

**11.** Procédé selon l'une quelconque des revendications précédentes, caractérisé en ce qu'on fait précipiter le ou les produits de la réaction du chlorure d'oxalyle avec le sel avant de chauffer le milieu réactionnel.

**12.** Procédé selon l'une quelconque des revendications précédentes, caractérisé en ce qu'on sépare, du milieu réactionnel, le ou les produits de la réaction du chlorure d'oxalyle avec le sel, puis en ce qu'on chauffe ce ou ces produits à une température comprise entre 30°C et 150°C, éventuellement en présence d'un milieu solvant.

**Patentansprüche**

**1.** Verfahren zur Herstellung von Acylisocyanaten der Formel $Z(CONCO)_n$,
in der bedeuten:
Z

- eine einwertige oder zweiwertige Gruppe, die ausgewählt ist unter
  - geradkettigen oder verzweigten, gesättigten oder ungesättigten und ggf. substituierten aliphatischen $C_{1-20}$-Gruppen,
  - gesättigten oder ungesättigten und ggf. substituierten cycloaliphatischen $C_{5-7}$-Gruppen,
  - geradkettigen oder verzweigten, gesättigten oder ungesättigten und ggf. substituierten araliphatischen $C_{7-20}$-Gruppen,
  - Phenyl, Phenylen, Naphthyl oder Naphthylen, die ggf. substituiert sind,
    und
  - gesättigten oder ungesättigten ggf. substituierten 5- oder 6-gliedrigen heterocyclischen Gruppen, die ein oder mehrere Heteroatome aufweisen, die unter Sauerstoff, Stickstoff und Schwefel ausgewählt sind,

  oder

- eine Gruppe RO-, -O-R-O- oder -R-O-,
  worin R eine einwertige oder zweiwertige Gruppe bedeutet, die ausgewählt ist unter
  - geradkettigen oder verzweigten, gesättigten oder ungesättigten und ggf. substituierten aliphatischen $C_{1-8}$-Gruppen,
  - gesättigten oder ungesättigten und ggf. substituierten cycloaliphatischen $C_{5-7}$-Gruppen,
  - geradkettigen oder verzweigten, gesättigten oder ungesättigten und ggf. substituierten araliphatischen $C_{7-11}$-Gruppen,
    und
  - Phenyl, Phenylen, Naphthyl oder Naphthylen, die ggf. substituiert sind,

und
n 1 oder 2,
gekennzeichnet durch

- Umsetzung von Oxalylchlorid mit einem Salz der Formel $Z(CONH_2)_n \cdot y(H_mX)$, in der bedeuten,

  Z und n dasselbe wie oben,
  m 1 oder 2,
  y den Wert $\frac{n}{m}$
  und
  X ein Halogenatom, $SO_4$ oder $HSO_4$,
  bei einer Temperatur von 15 bis 30 °C ohne Abkühlen des Reaktionsmediums und ohne Zusatz von Halogenwasserstoffsäure

  und

- anschließendes Erhitzen des Reaktionsmediums auf eine Temperatur von 30 bis 150 °C.

2. Verfahren nach Anspruch 1, dadurch gekennzeichnet, daß die Substituenten von Z ausgewählt sind unter Halogenatomen, über ein Sauerstoffatom gebundenen $C_{1-20}$-Kohlenwasserstoffgruppen und über ein Sauerstoffatom gebundenen halogenierten $C_{1-20}$-Kohlenwasserstoffgruppen sowie, wenn sich die Substituenten an einem aromatischen oder heteroaromatischen Ring befinden, unter $C_{1-20}$-Kohlenwasserstoffgruppen und halogenierten $C_{1-20}$-Kohlenwasserstoffgruppen sowie Nitro.

3. Verfahren nach Anspruch 1 oder 2, dadurch gekennzeichnet, daß X ein Chloratom bedeutet und m gleich 1 ist.

4. Verfahren nach einem der vorhergehenden Ansprüche, dadurch gekennzeichnet, daß es in einem inerten und aprotischen organischen Lösungsmittelmedium durchgeführt wird.

5. Verfahren nach Anspruch 4, dadurch gekennzeichnet, daß das Lösungsmittelmedium unter halogenierten oder nichthalogenierten aliphatischen Kohlenwasserstoffen, Cycloalkanen und aromatischen Kohlenwasserstoffen sowie Gemischen dieser Verbindungen ausgewählt ist.

6. Verfahren nach einem der vorhergehenden Ansprüche, dadurch gekennzeichent, daß Z eine ggf. substituierte aliphatische, cycloaliphatische oder araliphatische ungesättigte Gruppe darstellt.

7. Verfahren nach Anspruch 6, dadurch gekennzeichnet, daß Z die Gruppe $CH_2 = CH_2$- oder $CH_2$ =

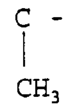

bedeutet.

8. Verfahren nach Anspruch 7, dadurch gekennzeichnet, daß als Lösungsmittelmedium ein Gemisch von o-Dichlorbenzol und einem niederen Alkan eingesetzt wird.

9. Verfahren nach einem der vorhergehenden Ansprüche, dadurch gekennzeichnet, daß das Oxalylchlorid in einer Menge von 1 bis 1,2 Äquivalent pro umzusetzende Salzfunktion eingesetzt wird.

10. Verfahren nach einem der vorhergehenden Ansprüche, dadurch gekennzeichnet, daß das Oxalylchlorid zu dem Salz zugegeben wird.

11. Verfahren nach einem der vorhergehenden Ansprüche, dadurch gekennzeichnet, daß das oder die Produkte der Umsetzung des Oxalylchlorids mit dem Salz vor dem Erwärmen des Reaktionsmediums ausgefällt werden.

12. Verfahren nach einem der vorhergehenden Ansprüche, dadurch gekennzeichnet, daß das oder die Produkte der Umsetzung des Oxalylchlorids mit dem Salz aus dem Reaktionsmedium abgetrennt werden und anschließend dieses Produkt oder diese Produkte, ggf. in Gegenwart eines Lösungsmittelmediums, auf eine Temperatur von 30 bis 150 °C erhitzt werden.

**Claims**

1. Method for preparing an acyl isocyanate having the formula $Z(CONCO)_n$ in which Z represents a mono- or divalent radical which is,

- a substituted or unsubstituted, saturated or unsaturated, linear or branched, aliphatic radical with $C_1$ to $C_{20}$,
- a substituted or unsubstituted, saturated or unsaturated, cycloaliphatic radical with $C_5$ to $C_7$,
- a substituted or unsubstituted, saturated or unsaturated, linear or branched, araliphatic radical with $C_7$ to $C_{20}$,
- a substituted or unsubstituted phenyl, phenylene, naphthyl or naphthylene radical,
- a substituted or unsubstituted, saturated or unsaturated, heterocyclic radical with 5 to 6 apices, having one or more heteroatoms selected from oxygen, nitrogen and sulphur,

or Z represents an RO-, -O-R-O- or -R-O- group in which R represents a mono- or divalent radical which is,

- a substituted or unsubstituted, saturated or unsaturated, linear or branched, aliphatic radical with $C_1$ to $C_8$,
- a substituted or unsubstituted, saturated or unsaturated, cycloaliphatic radical with $C_5$ to $C_7$,
- a substituted or unsubstituted, saturated or unsaturated, linear or branched, araliphatic radical with $C_7$ to $C_{11}$,
- a substituted or unsubstituted phenyl, phenylene, naphthyl or naphthylene radical,

and n represents the number 1 or 2, characterized in that oxalyl chloride is reacted with a salt having the formula $Z(CONH_2)_n$, $y(H_mX)$, in which Z and n have the previous meanings, m represents the number 1 or 2, y represents the value n/m and X represents a halogen atom, the $SO_4$ group or the $HSO_4$ group, at a temperature of between 15°C and 30°C or equal to these values, without cooling the reaction medium and without adding hydrohalogenic acid, and the reaction medium is then heated to a temperature of between 30°C and 150°C.

2. Method according to claim 1, characterized in that the substituents of Z are selected from halogen atoms and hydrocarbyloxy radicals and halogenohydrocarbyloxy radicals with $C_1$ to $C_{20}$ and in addition from the hydrocarbyl and halogenohydrocarbyl radicals with $C_1$ to $C_{20}$ and the nitro group when they are substituted on an aromatic ring or a heteroaromatic ring.

3. Method according to claim 1 or 2, characterized in that X represents a chlorine atom and m is equal to 1.

4. Method according to any one of the preceding claims, characterized in that it is carried out in an inert and aprotic organic solvent medium.

5. Method according to claim 4, characterized in that the solvent medium is selected from the group consisting of halogenated or non-halogenated aliphatic hydrocarbons cycloalkanes, aromatic hydrocarbons, and their mixtures.

6. Method according to any one of the preceding claims, characterized in that Z represents a substituted or unsubstituted, unsaturated aliphatic, cycloaliphatic or araliphatic radical.

7. Method according to claim 6, characterized in that Z represents the radical $CH_2 = CH_2$ - or $CH_2 =$

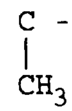

8. Method according to claim 7, characterized in that a mixture of o-dichlorobenzene and a light alkane is used as the solvent medium.

9. Method according to any one of the preceding claims, characterized in that 1 to 1.2 equivalents of oxalyl chloride are used per salt functional group to be reacted.

10. Method according to any one of the preceding claims, characterized in that oxalyl chloride is added to the salt.

11. Method according to any one of the preceding claims, characterized in that the product or products of the reaction between oxalyl chloride and the salt is or are precipitated before heating the reaction medium.

12. Method according to any one of the preceding claims, characterized in that the product or products of the reaction between oxalyl chloride and the salt is or are separated from the reaction medium, and this product or these products is or are then heated to a temperature of between 30°C and 150°C, optionally in the presence of a solvent medium.